# EUROPEAN PATENT SPECIFICATION

(11) **EP 3 528 792 B1**
(45) Date of publication and mention of the grant of the patent: **01.01.2025**
(21) Application number: 16798827.8
(22) Date of filing: 20.10.2016
(51) Int. Cl.: A61K 9/51, A61K 31/00

(54) **A METHOD OF MANUFACTURING A SOLUTION OF NSAID NANOPARTICLES**
VERFAHREN ZUR HERSTELLUNG EINER LÖSUNG AUS NSAID-NANOPARTIKELN
PROCÉDÉ DE FABRICATION D'UNE SOLUTION DE NANOPARTICULES D'AINS

(43) Date of publication of application: 28.08.2019
(73) Proprietor: Dukebox SP. Z O.O., 01-059 Warszawa (PL)
(72) Inventor: BANACH, Marcin, 32-100 Proszowice (PL)
(74) Representative: Krekora, Magdalena
(86) International application number: PCT/IB2016/095007
(87) International publication number: WO 2018/073632

(56) References cited:
- EP-A1- 0 644 755
- WO-A2-2013/132457
- CN-A- 103 655 501
- CN-C- 100 348 181
- US-A1- 2002 119 916
- US-A1- 2008 193 520
- US-A1- 2016 166 694
- US-B2- 8 445 545

## Description

### TECHNICAL FIELD

The object of the present invention is a method of manufacturing a solution of NSAID nanoparticles, in particular a propionic acid derivative NSAID.

### BACKGROUND ART

Non-steroidal anti-inflammatory drugs (NSAIDs) are drugs with analgesic, anti-inflammatory, antipyretic and osteoarthritis therapeutic activities and therefore are frequently used. NSAIDs are well defined group of compounds and comprise molecules derivatives of salicylate, acetic acid, propionic acid. Propionic acid derivative NSAIDs are ibuprofen, naproxen, ketoprofen, fenoprofen, benoxaprofen, suprofen, flurbiprofen, ibuproxam, alminoprofen, dexibuprofen, dexketoprofen and indoprofen. These molecules are used in the treatment or prevention of symptoms such as pain, inflammation and fever.

Many of the propionic acid derivative NSAIDs are poorly soluble in water and other solvents like for instance ibuprofen. Poor solubility of active substances affects negatively their bioavailability. However in accordance with the Ostwald-Freundlich equation reduction of particles size increase significantly their solubility. Therefore formation of NSAID particles, in particular ibuprofen particles, whose maximum size is equal or smaller than 100 nm, would significantly improve pharmacological activity of those active substances.

European patent application EP0644755 discloses a method of preparing surface modified nanoparticles consisting of a crystalline NSAID having a surface modifier adsorbed on the surface thereof in an amount sufficient to maintain an average particle size of less than 400 nm, the method comprising the steps of dispersing the NSAID in a liquid dispersion medium and wet grinding the NSAID in the presence of rigid grinding media, to an effective average particle size of less than about 400 nm, wherein the pH of said medium is maintained within the range of from 2 to 6 during said wet grinding, with the proviso that the NSAID is other than ibuprofen. The preferred liquid dispersion medium is water. However, the invention can be practiced with other liquid media in which the NSAID is poorly soluble and dispersible including, for example, aqueous salt solutions, safflower oil and solvents such as ethanol, t-butanol, hexane and glycol.

Patent application CN103655501 discloses a preparation method of nano ibuprofen dry powder and tablets thereof. The preparation method comprises the following steps of: firstly dissolving ibuprofen in an organic solvent, then dissolving multiple pharmaceutical adjuvants into water, carrying out spraying and drying on slurry formed by mixing the two types of solution under proper conditions at room temperature, successfully preparing nano ibuprofen powder with the water-soluble particle diameter being 20-200nm, and mixing and pressing the dry powder and proper adjuvants into the tablets.

Patent CN100348181 discloses a method of preparation of injectable formulations of water-dispersible ibuprofen nanoparticles where nanoparticles are stabilized with polysaccharides.

Patent specification US8445545 discloses a process for preparing a stable, water-soluble ibuprofen composition having a solubility range of 0.0001% to 99.9999%, comprising the steps of:
a) dissolving ibuprofen USP grade powder in a succession manner according to HPUS with a dissolution solution having water, alcohol, glycerin or a combination thereof thereby forming an ibuprofen solution;
b) transferring said ibuprofen solution to a vacuum equipped vessel having a high shear mixer mounted thereon;
c) mixing said ibuprofen solution in said vacuum equipped vessel having a high shear mixer mounted thereon for 10-30 minutes;
d) reducing the particle size of said ibuprofen solution to less than or equal to 500 nanometers;
e) allowing the mixture from step c) to rest for 10-20 minutes; and
f) equilibrating the product for approximately 24 hours.

Patent application US20020119916 discloses a method of making nanoparticles of a substantially water insoluble material, e.g. methotrexate, progesterone, testosterone, prednisolone, and ibuprofen comprising:
(a) preparing an emulsion system, having a dispersed phase and a continuous phase; the dispersed phase comprises globules containing said material within the continuous phase in the presence of a surfactant;
(b) diluting the emulsion by addition of a liquid that is miscible with the dispersed phase and the continuous phase, in an amount effective to dissociate said emulsion, thereby producing a uniform liquid phase in which nanoparticles of said material are suspended, said nanoparticles having an average particle size equal to or less than the globule size of said dispersed phase, and, optionally,
(c) separating said nanoparticles from said uniform liquid phase.

### DISCLOSURE OF THE INVENTION

The method in accordance with the present invention and defined by the appended claims solves the problem of preparation of solutions of a propionic acid derivative NSAID, and thus improving their bioavailability and efficacy. Propionic acid derivative NSAIDs are ibuprofen, naproxen, ketoprofen, fenoprofen, benoxaprofen, suprofen, flurbiprofen, ibuproxam, alminoprofen, dexibuprofen, dexketoprofen and indoprofen.

A method of manufacturing a solution of NSAID nanoparticles of the present invention consists in that, a propionic acid derivative NSAID is dissolved in an organic solvent, and separately at least one dispersing agents is dissolved in water, then the organic solution is added to the aqueous solution and the obtained mixture is homogenised until homogenous solution is obtained. The method leads to obtaining dispersed nanoparticles of the a propionic acid derivative NSAID, whose average diameter is equal to about 100 nm or less. Obtained solution is a final product, that may be administered to patients as a medicinal product, or may be used as a starting material for manufacture of medicinal products. The solution possesses enhanced bioavailability and is very stable as the nanoparticles do not aggregate. The method is technologically simple, and safe.

Preferably the NSAID is ibuprofen.

The NSAID in dissolved in ethanol.

The solution of NSAID in ethanol has concentration from 70 to 1050 mg/dm³, preferably about 360 g/dm³.

Preferably the concentration of organic solvent in the obtained mixture is not higher than 20%.

Preferably dissolution of dispersing agents in water is carried out in a temperature non higher than 90°C.

The dispersing agents are chosen from a group consisting of lecithin, gelatine and starch.

The aqueous solution of dispersing agents contains lecithin in concentration from 25,0 to 66,67 g/dm³ and gelatine in concentration from 1,67 to 6,67 g/dm³ or the aqueous solution of dispersing agents contains lecithin in concentration from 23,22 to 66,67 g/dm³ and starch in concentration from 3,33 to 33,33 g/dm³.

Preferably the aqueous solution of dispersing agents contains potassium sorbate.

Preferably the concentration of potassium sorbate is from 1,33 to 33,33 g/dm³.

Preferably the volume ratio of organic solution of NSAID to the aqueous solution of dispersing agents is from 0,1 to 0,2.

Preferably the mixture of organic solution of NSAID and the aqueous solution is homogenised through the use of ultrasonic frequencies.

Preferably the strength of ultrasound is from 500 to 600 W.

Preferably the homogenization process is carried out from 1 to 15 minutes.

Preferably the ultrasonication is carried out in flow system or in batch system.

The present invention refers also to a solution of NSAID nanoparticles obtained by method according to the invention.

While the organic solution of NSAID, in particular alcoholic solution of ibuprofen, is introduced into aqueous solution of other ingredients, it is precipitated. Sonication process enables obtaining of particles having nanometric size. This size is preserved as a result of stabilisation activity of lecithin, gelatine and starch. Sonication may be carried out in a batch reactor or in a flow system, each time supplied with a sonication kit having appropriate power - preferably from 500 to 600 W.

### BEST MODE OF CARRYING OUT THE INVENTION

The following examples illustrate the invention without setting or delineating its limits.

### Example 1

### Obtaining of a solution gelatine/lecithin/ibuprofen

Ethanol solution of ibuprofen was prepared by adding 18 g of ibuprofen to 50 cm³ ethanol (99,6%) and aqueous solution of gelatine and lecithin by adding 10 g of lecithin and 2 g of gelatine to up to 300 cm³ of water. Gelatine and lecithin were dissolved in the temperature of about 50°C. Then the solution of ibuprofen was added to the solution of gelatine and lecithin. The volume ratio of both solution was equal to 0.167. Obtained solution underwent sonication. The process of sonication was carried out for 5 minutes at a power of 600W.

The Malvern Zetasizer apparatus using method of dynamic light scattering (DLS) was utilized to measure the average size of particles. Potential zeta was also measured. The solution of ibuprofen nanoparticles obtained had concentration of about 55 mg/cm³ and the average size of nanoparticles was 66 nm. Potential zeta was equal to -32,4 mV. The absolute value of the electrokinetic (zeta) potential was above 30 mV, what confirms high stability of the solution.

### Example 2

### Obtaining of a solution starch/lecithin/potassium sorbate/ibuprofen

Ethanol solution of ibuprofen was prepared by adding 18 g of ibuprofen to 50 cm³ ethanol (99,6%) and aqueous solution of gelatine and lecithin by adding 10 g of lecithin and 2 g of starch to up to 300 cm³ of water. Starch and lecithin were dissolved in the temperature of about 70°C, and then 0,8 g of potassium sorbate was added to the solution, and the whole was mixed. Afterwards the solution of ibuprofen was added to the solution of starch, lecithin, and potassium sorbate. Obtained solution was treated with ultrasounds for 3 minutes at a power of 500W.

The Malvern Zetasizer apparatus using method of dynamic light scattering (DLS) was utilized to measure the average size of particles. Potential zeta was also measured. The average size of nanoparticles was 105 nm. Potential zeta was equal to -38,8 mV. The size of particles was then measured after 24 hours, and was equal to 96nm. Lack of significant changes of the average nanoparticles size, as well as the absolute value of the electrokinetic (zeta) potential above 30 mV, confirm high stability of the solution.

### Example 3

### Obtaining of a solution gelatine/lecithin/naproxen

Ethanol solution of naproxen was prepared by adding 18 g of naproxen to 50 cm³ ethanol (99,6%) and aqueous solution of gelatine and lecithin by adding 15 g of lecithin and 1 g of gelatine to up to 300 cm³ of water. Gelatine and lecithin were dissolved in the temperature of about 70°C. Then the solution of naproxen was added to the solution of gelatine and lecithin. The volume ratio of both solution was equal to 0.18. Obtained solution underwent sonication. The process of sonication was carried out for 5 minutes at a power of 600W.

The Malvern Zetasizer apparatus using method of dynamic light scattering (DLS) was utilized to measure the average size of particles. Potential zeta was also measured. The solution of naproxen nanoparticles obtained had concentration of about 55 mg/cm³ and the average size of nanoparticles was 86 nm. Potential zeta was equal to -30,2 mV. The absolute value of the electrokinetic (zeta) potential was above 30 mV, what confirms high stability of the solution.

### Example 4

### Obtaining of a solution starch/lecithin/potassium sorbate/ketoprofen

Ethanol solution of ketoprofen was prepared by adding 18 g of ketoprofen to 50 cm³ ethanol (99,6%) and aqueous solution of gelatine and lecithin by adding 20 g of lecithin and 2 g of starch to up to 300 cm³ of water. Starch and lecithin were dissolved in the temperature of about 70°C, and then 0,8 g of potassium sorbate was added to the solution, and the whole was mixed. Afterwards the solution of ketoprofen was added to the solution of starch, lecithin, and potassium sorbate. Obtained solution was treated with ultrasounds for 5 minutes at a power of 600W.

The Malvern Zetasizer apparatus using method of dynamic light scattering (DLS) was utilized to measure the average size of particles. Potential zeta was also measured. The average size of nanoparticles was 135 nm. Potential zeta was equal to -31,4 mV. The absolute value of the electrokinetic (zeta) potential above 30 mV confirms high stability of the solution.

## Claims

1. A method of manufacturing a solution of NSAID nanoparticles, **characterised in that**, a propionic acid derivative NSAID is dissolved in ethanol, and at least one dispersing agents chosen from a group consisting of lecithin, gelatin and/or starch is dissolved in water to form the aqueous solution of dispersing agents which contains lecithin in concentration from 25,0 to 66,67 g/dm³ and gelatin in concentration from 1,67 to 6,67 g/dm³ or to form the aqueous solution of dispersing agents which contains lecithin in concentration from 23,22 to 66,67 g/dm³ and starch in concentration from 3,33 to 33,33 g/dm³, then the organic solution is added to the aqueous solution and the obtained mixture is homogenised until homogenous solution is obtained.

2. A method according to claim 1, **characterized in that** the NSAID is ibuprofen.

3. A method according to claim 1 or 2, **characterized in that**, solution of NSAID in ethanol has concentration of from 70 to 1050 g/dm³.

4. A method according to any of the preceding claims, **characterized in that**, the concentration of organic solvent in the obtained mixture is not higher than 20%.

5. A method according to any of the preceding claims, **characterized in that**, dissolution of dispersing agents in water is carried out in a temperature non higher than 90°C.

6. A method according to claim any of the preceding claim, **characterized in that**, the aqueous solution of dispersing agents contains potassium sorbate.

7. A method according to claim 8, **characterized in that**, the concentration of potassium sorbate is from 1,33 to 33,33 g/dm³.

8. A method according to any of the preceding claims, **characterized in that**, the volume ratio of organic solution of NSAID to the aqueous solution of dispersing agents is from 0,1 to 0,2.

9. A method according to any of the preceding claims, **characterized in that**, the mixture of organic solution of NSAID and the aqueous solution is homogenised through the use of ultrasonic frequencies.

10. A method according to claim 9, **characterized in that**, the strength of ultrasound is from 500 to 600 W.

11. A method according to claim 9 or 10, **characterized in that**, the homogenization process is carried out from 1 to 15 minutes.

12. A method according to claim 9 or 10 or 11, **characterized in that**, the ultrasonication is carried out in flow system or in batch system.

13. A solution of NSAID nanoparticles obtained by method according to any of the preceding claims.

## Patentansprüche

1. Verfahren zur Herstellung einer Lösung von NSAID-Nanopartikeln, **dadurch gekennzeichnet, dass** ein Propionsäurederivat-NSAID in Ethanol gelöst wird und mindestens ein Dispergiermittel, das aus einer Gruppe ausgewählt wird, die aus Lecithin, Gelatine und/oder Stärke besteht, in Wasser gelöst wird, um die wässrige Lösung von Dispergiermitteln zu bilden, die Lecithin in einer Konzentration von 25,0 bis 66,67 g/dm³ und Gelatine in einer Konzentration von 1,67 bis 6,67 g/dm³ oder zur Bildung einer wässrigen Lösung von Dispergiermitteln, die Lecithin in einer Konzentration von 23,22 bis 66,67 g/dm³ und Stärke in einer Konzentration von 3,33 bis 33,33 g/dm³ enthält, gelöst wird, dann die organische Lösung zu der wässrigen Lösung gegeben wird und die erhaltene Mischung homogenisiert wird, bis eine homogene Lösung erhalten wird.

2. Verfahren nach Anspruch 1, **dadurch gekennzeichnet, dass** das NSAID Ibuprofen ist.

3. Verfahren nach Anspruch 1 oder 2, **dadurch gekennzeichnet, dass** die Lösung des NSAID in Ethanol eine Konzentration von 70 bis 1050 g/dm³ aufweist.

4. Verfahren nach einem der vorstehenden Ansprüche, **dadurch gekennzeichnet, dass** die Konzentration des organischen Lösungsmittels in der erhaltenen Mischung nicht höher als 20 % ist.

5. Verfahren nach einem der vorhergehenden Ansprüche, **dadurch gekennzeichnet, dass** die Auflösung von Dispergiermitteln in Wasser bei einer Temperatur von nicht mehr als 90 °C durchgeführt wird.

6. Verfahren nach einem der vorhergehenden Ansprüche, **dadurch gekennzeichnet, dass** die wässrige Lösung der Dispergiermittel Kaliumsorbat enthält.

7. Verfahren nach Anspruch 8, **dadurch gekennzeichnet, dass** die Konzentration an Kaliumsorbat 1,33 bis 33,33 g/dm³ beträgt.

8. Verfahren nach einem der vorstehenden Ansprüche, **dadurch gekennzeichnet, dass** das Volumenverhältnis der organischen Lösung des NSAID zur wässrigen Lösung der Dispergiermittel 0,1 bis 0,2 beträgt.

9. Verfahren nach einem der vorstehenden Ansprüche, **dadurch gekennzeichnet, dass** die Mischung aus der organischen Lösung des NSAID und der wässrigen Lösung durch die Verwendung von Ultraschallfrequenzen homogenisiert wird.

10. Verfahren nach Anspruch 9, **dadurch gekennzeichnet, dass** die Ultraschallstärke 500 bis 600 W beträgt.

11. Verfahren nach Anspruch 9 oder 10, **dadurch gekennzeichnet, dass** der Homogenisierungsprozess 1 bis 15 Minuten dauert.

12. Verfahren nach Anspruch 9 oder 10 oder 11, **dadurch gekennzeichnet, dass** die Ultraschallbehandlung in einem Durchflusssystem oder in einem Chargensystem durchgeführt wird.

13. Lösung von NSAID-Nanopartikeln, die durch ein Verfahren gemäß einem der vorstehenden Ansprüche erhalten wurde.

## Revendications

1. Une méthode de fabrication d'une solution de nanoparticules d'AINS, **caractérisé en ce qu'**un AINS dérivé de l'acide propionique est dissous dans de l'éthanol, et au moins un agent dispersant choisi dans le groupe constitué de la lécithine, de la gélatine et/ou de l'amidon est dissous dans l'eau pour former la solution aqueuse d'agents dispersants qui contient de la lécithine en concentration de 25,0 à 66,67 g/dm³et de la gélatine en concentration de 1,67 à 6,67 g/dm³ ou pour former la solution aqueuse d'agents dispersants qui contient de la lécithine en concentration de 23,22 à 66,67 g/dm³ et de l'amidon en concentration de 3,33 à 33,33 g/dm³, puis la solution organique est ajoutée à la solution aqueuse et le mélange obtenu est homogénéisé jusqu'à obtention d'une solution homogène.

2. Une méthode selon la revendication 1, **caractérisée en ce que** l'AINS est l'ibuprofène.

3. Une méthode selon la revendication 1 ou 2, **caractérisée en ce que** la solution d'AINS dans l'éthanol a une concentration de 70 à 1050 g/dm³.

4. Une méthode selon l'une quelconque des revendications précédentes, **caractérisée en ce que** la concentration en solvant organique dans le mélange obtenu n'est pas supérieure à 20 %.

5. Une méthode selon l'une quelconque des revendications précédentes, **caractérisée en ce que** la dissolution des agents dispersants dans l'eau est effectuée à une température pas plus élevée que 90°C.

6. Une méthode selon l'une quelconque des revendications précédentes, **caractérisée en ce que** la solution aqueuse d'agents dispersants contient du sorbate de potassium.

7. Une méthode selon la revendication 8, **caractérisée en ce que** la concentration en sorbate de potassium est de 1,33 à 33,33 g/dm³.

8. Une méthode selon l'une quelconque des revendications précédentes, **caractérisée en ce que** le rapport volumique de la solution organique d'AINS à la solution aqueuse d'agents dispersants est de 0,1 à 0,2.

9. Une méthode selon l'une quelconque des revendications précédentes, **caractérisée en ce que** le mélange de solution organique d'AINS et de solution aqueuse est homogénéisé grâce à l'utilisation de fréquences ultrasonores.

10. Une méthode selon la revendication 9, **caractérisée en ce que** la puissance des ultrasons est de 500 à 600 W.

11. Une méthode selon la revendication 9 ou 10, **caractérisée en ce que** le processus d'homogénéisation est réalisé de 1 à 15 minutes.

12. Une méthode selon la revendication 9 ou 10 ou 11, **caractérisée en ce que** l'ultrasonication est réalisée en système d'écoulement ou en système par lots.

13. Solution de nanoparticules d'AINS obtenue par une méthode selon l'une quelconque des revendications précédentes.
